# EUROPEAN PATENT APPLICATION

(11) **EP 3 076 181 A1**
(43) Date of publication of application: **05.10.2016**
(21) Application number: 15305474.7
(22) Date of filing: 31.03.2015
(51) Int. Cl.: G01N 33/68, G06F 19/00

(54) **ATOMIC STRUCTURE OF THE HUMAN 80S RIBOSOME**

(71) Applicant: CENTRE NATIONAL DE LA RECHERCHE SCIENTIFIQUE -CNRS-, 75794 Paris Cedex 16 (FR); INSERM - Institut National de la Santé et de la Recherche Médicale, 75654 Paris Cedex (FR); Université de Strasbourg, 67000 Strasbourg (FR)
(72) Inventor: Khatter, Heena, 110017 NEW DELHI (IN); Myasnikov, Alexander, 67100 STRASBOURG (FR); Natchiar, Kundhavai, 67400 ILLKRICH (FR); Klaholz, Bruno, 67400 ILLKIRCH (FR)
(74) Representative: Novagraaf Technologies

(57) **Abstract**

The present invention relates to a method for providing the structure of the human ribosome 80S at an atomic level derived from high-resolution single particle cryo electron microscopy (cryo-EM) and atomic model building, the atomic structure of the human ribosome obtained by said method and uses thereof.

## Description

### Field of the invention

The present invention relates to a method for providing the structure of the human ribosome 80S at the atomic level derived from high-resolution single particle cryo electron microscopy (cryo-EM) and atomic model building, the atomic structure of the human ribosome obtained by said method and uses thereof.

The present invention applies in the therapeutic field, particularly for studying molecular mechanisms and exploring medical applications in the ribosome field.

In the description below, the references between square brackets ([ ]) refer to the list of references presented at the end of the text.

### Background

Ribosomes form the hub of translation machineries across all kingdoms of life, synthesizing proteins using an mRNA template. An interesting concept that has emerged from previous structures is the layered evolution of the ribosome with a conserved core existing across all species, comprising the catalytically active peptidyl transferase centre (PTC) and GTPase (guanosine-triphosphatase) associated centre located in the large ribosomal subunit and the decoding centre (DC) in the small subunit. Hence, the ribosomes of multicellular eukaryotes are particularly complex and contain additional ribosomal RNA (rRNA) components, termed expansion segments (ES), and additional protein moieties as compared to their prokaryotic counterparts.

The human ribosome has a molecular weight of 4.3 MDa, with the large subunit (60S) consisting of 28S, 5S and 5.8S rRNA and 47 proteins, while the small subunit (40S) possesses a single rRNA chain, 18S, associated with 33 proteins. Until 2013, structures of only unicellular eukaryotic ribosomes were known to near-atomic resolution solved using X-ray crystallography, [1-3] while the ribosome structures from higher eukaryotes had been studied at relatively medium resolution using cryo electron microscopy (cryo-EM) [4-7]. The structures of mammalian cytoplasmic ribosomes have provided location and folds of all eukaryote-specific components.

A structure of the human ribosome at an average resolution of ∼5.4-6.0 Å recently gave first insights [6]. However, an atomic structure of the human ribosome is still missing because this would require structural data closer to the 3.0 Å resolution range. Another important aspect is the presence of Stm1-like inactivating factors in the mRNA channel in the previously reported structure of the human ribosome [6] as was also observed for the yeast ribosome [1], a property which may make the biochemical assembly of functionally active complexes difficult. It is also of primary importance to have an atomic structure of the human ribosome considering its medical implications as a target for various human diseases, including cancer because of the elevated protein synthesis rate in cancer cells. Furthermore, it is a central target in the treatment of infectious diseases and is of special interest with regards to the growing problems of antibiotic resistance and side-effects caused by drug therapies, the latter being related with a limited specificity between bacterial and human ribosomes.

Over the past year, structure determination in the 3-4 Å resolution range of asymmetric objects such as the ribosome by single particle cryo-EM has become feasible as observed in [8-11], owing in part to remarkably improved electron detectors, [12-14]. Nevertheless, the potential of cryo-EM associated atomic model building and refinement procedures as used in the field of X-ray crystallography is not yet fully exploited especially in terms of structure refinement of large macromolecular complexes.

There is therefore a real need for a method overcoming the shortcomings, disadvantages and obstacles of prior art, particularly for a method providing the human ribosome structure at the atomic level, to reveal atomic details that would be highly relevant with regards to human diseases of various kinds associated with protein synthesis deregulation (bacterial, parasite and viral infections, cancer and others to be identified in relationship with the human ribosome target).

### Summary of the invention

While ribosome structures from various species are known at the atomic level, that of the human ribosome remained a challenge to address.

Rather than using X-ray crystallography which has been a standard tool in structural biology for the structure determination of nucleoprotein complexes in order to derive experimentally derived models, the inventors have now unexpectedly succeeded in determining the near-atomic structure of the human ribosome using high-resolution electron microscopy and a direct electron detector (Complementary Metal Oxide Semiconductor (CMOS) detector with a back-thinned Direct Detection Device (DDD) sensor) combined with the recent developments in image processing (dose fractionation and movie processing) and 3D reconstruction that were specifically optimized for the human ribosome (*e.g.* 3 frames film processing rather than 7-17). The high-resolution cryo-EM map allowed building a detailed atomic model which was refined against the cryo-EM data.

The detailed structure of the human 80S ribosome obtained by the method of the present invention provides unprecedented insights into ribosomal RNAs and proteins down to the level of individual residues, inter-subunit contacts, interactions with the tRNA present in the exit (E) site, and which can be further exploited for the molecular description of antibiotic binding pockets and other potential drug pockets. The structure has an average resolution of 3.6 Å and reaches 2.9 Å resolution and better (2.4 A) in the most stable regions and thus provides unprecedented insights into rRNA entities and amino acid side-chains, notably of the tRNA binding sites and specific molecular interactions with the exit site tRNA. It reveals atomic details of the subunit interface which is seen to strongly remodel upon rotational movements of the ribosomal subunits. Furthermore, the structure paves the way for analysing antibiotic resistance and diseases associated with deregulated protein synthesis.

Thus an object of the present invention is the use of a 3D structural representation at atomic resolution of the human 80S ribosome obtained by a method comprising the steps of:
**a.** providing highly homogenous human 80S ribosomes;
**b.** characterizing the structural representation at atomic resolution of the human 80S ribosome using a high-resolution cryo electron microscope equipped with a high-sensitivity direct electron detector;
**c.** high-resolution image processing and 3D reconstruction of the single particle images obtained in step b) with a resolution in the range of 1.5-3.6 Å, preferably of 1.8-3.2 Å in the core structure;
in a drug design method or a screening method.

Another object of the present invention is a method of using the 3D structural representation at atomic resolution of the human 80S ribosome obtained as defined above, to identify a molecule of interest that can selectively bind said ribosome.

According to a particular embodiment of the claimed invention, the molecule of interest is chosen from the group consisting in anti-cancer compounds, anti-infectious compounds (against bacteria, parasites, viruses) such as antibiotics and anti-viral agents, and ribosome-associated cellular and extra-cellular protein and RNA factors such as tRNA's and viral mRNA.

Another object of the preset invention is a screening method for the identification of a compound that selectively binds the human 80S ribosome, comprising the steps of :
**a)** providing highly homogenous human 80S ribosomes;
**b)** providing a test compound;
**c)** contacting the human 80S ribosomes with the test compound;
**d)** characterizing the structural representation at atomic resolution of the complex formed in step c) using a high-resolution cryo electron microscope equipped with a high-sensitivity direct electron detector;
**e)** high-resolution image processing and 3D reconstruction of the single particle images obtained in step d) with a resolution in the range of 1.5-3.6 Å, preferably of 1.8-3.2 Å.

According to a particular embodiment of the claimed invention, the screening method of the invention may further comprise a step of f) assaying the interaction between the test compound and the human 80S ribosome.

According to a particular embodiment of the claimed invention, the test compound is chosen from the group consisting in anti-cancer compounds, antibiotics, antiviral agents and tRNA, protein factors (ribosomal initiation, elongation and termination factors), mRNA, viral RNA and miRNA, cellular and extra-cellular proteins and RNAs that bind to the human ribosome. For example, clinical antibiotics, 1-metronidazole, Lincomycin, Linezolid, edeine, hygromycin B, 13-deoxytedanolide, blasticidin, lactimidomycin, clarithromycin, pristinamycin, sulfamethoxazole, trimethoprim, amoxicillin, clavulanic acid, tetracycline derivatives, doxycycline, minocycline, tigecycline, all antibiotics cited in doi: 10.1016/j.cell.2009.08.009 and doi: 10.1016/j.cell.2009.08.001 published in Cell in 2009 [57, 58], anti-viral agents, anti-cancer agents, and fragments (for fragment screening and chemical synthesis of lead compounds) of chemical libraries such as those of the Preswick library.

Another object of the present invention is a computer-based method for identifying a ligand for the human 80S ribosome, comprising the steps of:
**a)** providing a 3D structural representation of the human 80S ribosome obtained as defined above; and
**b)** using the computer to apply structure-based drug design techniques to the structural representation.

Structure-based drug design techniques can be applied to the structural representation of the human 80S ribosome in order to identify, design or synthesize compounds that interact with it. A variety of suitable techniques are available to the skilled person, for example computer-assisted in silico screening [Sybyl by Tripos; Molecular Operating Environment (MOE) by Chemical Computing Group; MCSS, available from Molecular Simulations, Burlington, Mass [62]; AUTODOCK, available from Scripps Research Institute, La Jolla, Calif [63]); GOLD for protein-ligand docking].

According to a particular embodiment of the claimed invention, the computer-based method may further comprise at least one step of docking. By "docking" is meant according to the present invention, alignment of the 3D structures of two or more molecules to predict the conformation of a complex formed from said molecules. According to the present invention, molecules are docked with the human 80S ribosome structure to assess their ability to interact with it. Docking can be carried out by either geometric shape electrostatics, size and hydrophobicity matching of the ligand and its receptor, for example using an algorithm, or by minimizing the energy of interaction. Suitable docking algorithms include, for example that of Gold et al. (J. Mol. Biol., 267(3): 727-748, 1997) [59].

According to a particular embodiment of the claimed invention, the docking step screens members of a structural library of potential ligands and predicts interactions of in silico modified ligands.

Suitable structural libraries include, for example, the national Cancer Institute database (NCI), the above mentioned antibiotics and anti-viral agents, and ligands from the Preswick chemical ligand library.

According to a particular embodiment of the claimed invention, the computer-based method may further comprise a step of c) providing a compound identified by said-structure-based drug design techniques; and **d)** contacting said compound with the human 80S ribosome and assaying the interaction between them.

Another object of the present invention is a method for obtaining a 3D structural representation at atomic resolution of the human 80S ribosome, said method comprising the steps of:
**a)** providing highly homogenous human 80S ribosomes;
**b)** characterizing the structural representation at atomic resolution of the human 80S ribosome using a high-resolution cryo electron microscope equipped with a high-sensitivity direct electron detector;
**c)** high-resolution image processing and 3D reconstruction of the images obtained in step b) with a resolution in the range of 1.5-3.6 Å, preferably of 1.8-3.2 Å.

Another object of the present invention is a 3D structural representation at atomic resolution of the human 80S ribosome having a resolution of at least 3 Å and better, preferably having a resolution of 1.5-3.6 Å, more preferably of 1.8-3.2 Å, obtained by the method of the invention.

Another object of the present invention is the use of the 3D structural representation at atomic resolution of the human 80S ribosome of the invention into a drug design method or a screening method.

The present invention provides the first high-resolution structure of the human 80S ribosome without the requirement or use of crystallization, *i.e.* by using a simplified procedure over previously described method, while obtaining a better resolution and allowing structure determination of complexes.The present invention thus addresses a major challenge in this field where said high-resolution structure of the human 80S ribosome can be considered an ultimate goal of eukaryotic ribosome structure studies. This structure provides unprecedented insights into rRNA and amino acid residues including detailed side-chains positions and conformations and allow the comprehensive analysis of molecular recognition of ligands as listed above. These insights reveal the basis of molecular recognition that occurs through hydrogen bonding between ribosomal RNA and/or protein moieties within the entire ribosome structure, at the level of the exit site tRNA and within the subunit interface which is seen to remodel upon rotation of the ribosomal subunits. The comparison of the pre- and post-translocation states illustrates how inter-subunit bridges have a dramatic impact on the dynamics of the human ribosome and provides atomic details into subunit interface rearrangements that have never been seen before to this level of detail. While the present ribosome complex contains only an E-site tRNA for which detailed interactions are described here, its structure constitutes a reference for future functional complexes with ribosomal factors such as mRNA, tRNA and associated protein factors, either directly involved in the translation mechanism or implicated in general translation regulation. Biochemically, the present human 80S ribosome samples will be better suited for forming functional complexes because they do not contain any hibernation or starvation factors such as Stm1 [1] or serpine 1 mRNA-binding protein 1 (SERBP1) [6] located within the path of the mRNA in previous structures.

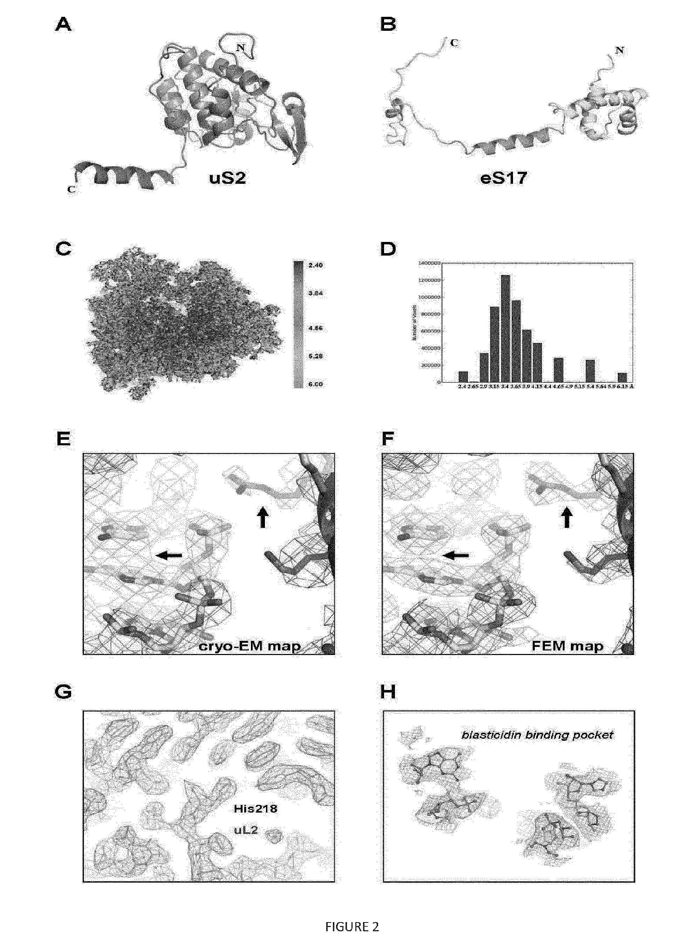

Another important aspect is that for drug design purposes it is crucial to work with the human target rather than with that of related model systems because of unpredictable effects such as second layer residues around ligand-binding pockets which can modify the interaction pattern of the primary contacting residues or open new pockets by conformational changes of side-chains. The structure provides a valuable repertoire and molecular description of antibiotic binding pockets (see as an example the antibiotic binding pocket displayed in **Fig. 2H****)** and other new potentially druggable ligand binding pockets which can serve as a basis for structure-guided computer-assisted drug design, *in silico* screening and structure determination of complexes with pharmaceutically relevant ligands. In this respect several major applications can already be considered: (i) try to reduce side-effects of current antibiotics by improving drug specificity to better distinguish between bacterial and human ribosomes, (ii) develop new anti-cancer agents that target the human ribosome to specifically reduce the elevated protein synthesis rate of cancer cells that makes them a primary target over normally growing cells, and (iii) develop anti-viral agents to block the binding of viral mRNA's to the human ribosome. The present high-resolution human ribosome structure thus constitutes a solid basis for new opportunities in drug developments directly relevant for human health.

Other advantages may also occur to a skilled person in the art upon reading the examples below, illustrated by the attached figures given for illustrative purposes.

### Brief description of the drawings

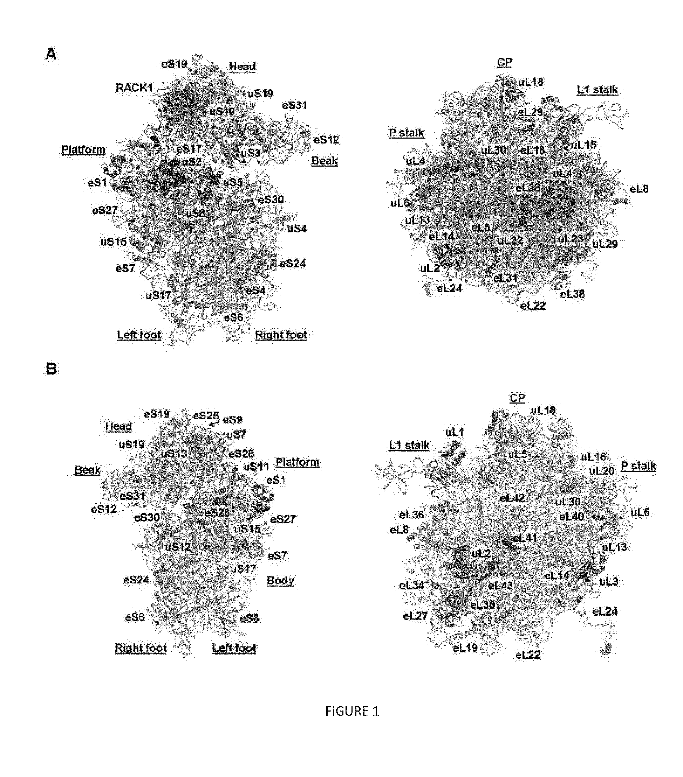

- Figure 1 represents the overall structure of the human 80S ribosome. **(A, B)** global views of the 80S human ribosome from the solvent sides of the 60S and 40S subunits (top lane) and from the inter-subunits interfaces with ribosomal proteins indicated on the atomic model (backbone representation).
- Figure 2 represents the structure of some ribosomal proteins and local resolution. **(A, B)** structure of ribosomal proteins uS2 and eS17 **(C, D)** Local resolution estimation of the cryo-EM map (central section perpendicular to the ribosomal subunit interface) and histogram distribution of resolution values **(E, F)** Comparison of cryo-EM and FEM maps. The bold arrows highlight some of the regions with improved density **(G)** Example of the electron density maps obtained by combining amplitudes derived from the cryo-EM map and phases calculated from the iteratively refined atomic model (Phenix map calculation), illustrating the interactions of ribosomal protein uL2 with 28S rRNA **(H)** Example of the definition of side-chain residues forming a ligand-binding pocket (here blasticidin, a non-specific antibiotic) as required for structure-guided drug design purposes.

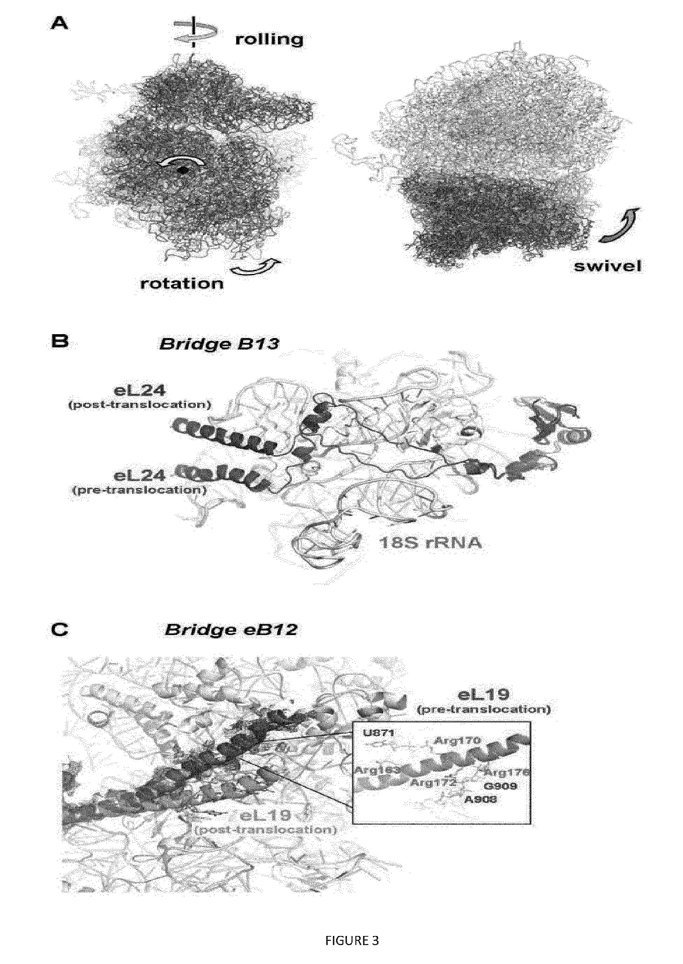

- Figure 3 represents conformational changes of the human ribosome. **(A)** Comparison of the human ribosome structure in the pre-translocation state (present study, 40S subunit in pink, 60S subunit in grey) and in the post-translocation state (when factor eEF2 is present, 40S subunit in cyan, porcine ribosome), revealing three types of movements (subunit rotation around an axis perpendicular to the subunit interface, subunit rolling around a vertical axis, and swivel movement of the 40S subunit head) **(B, C)** Illustration of large conformational changes of protein eL24 and eL19 occurring upon 40S subunit rotation. The C-terminal helix of eL24 is relocated to interact with a different region of the 18S rRNA. eL19 undergoes a structural transition from a straight (green, post-translocation state [6, 8]) to a bent protein helix (pink, pre-translocation state, present study; cryo-EM density with atomic model without side-chains for clarity). The inset shows the residues involved in this protein-RNA contact.

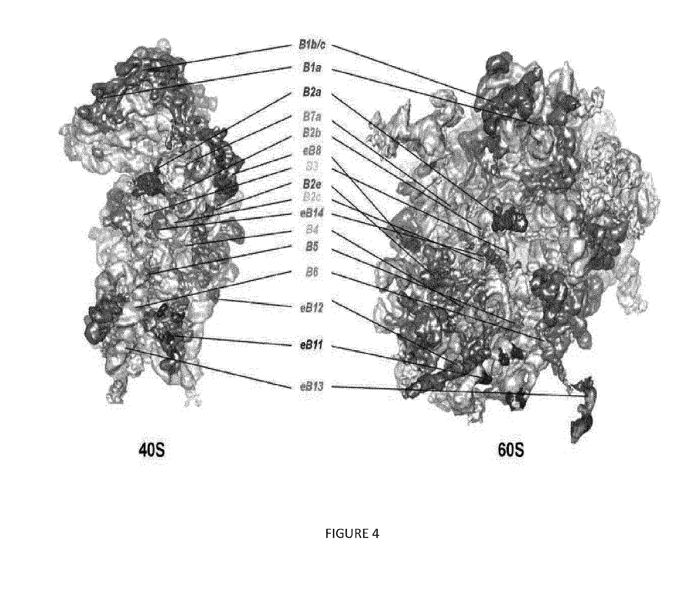

- Figure 4 represents ribosomal inter-subunit bridges. Annotation of the inter-subunit bridges at the interface of the 40S and 60S subunits (rRNA in light grey, ribosomal proteins in dark grey, color-coded bridges are indicated).

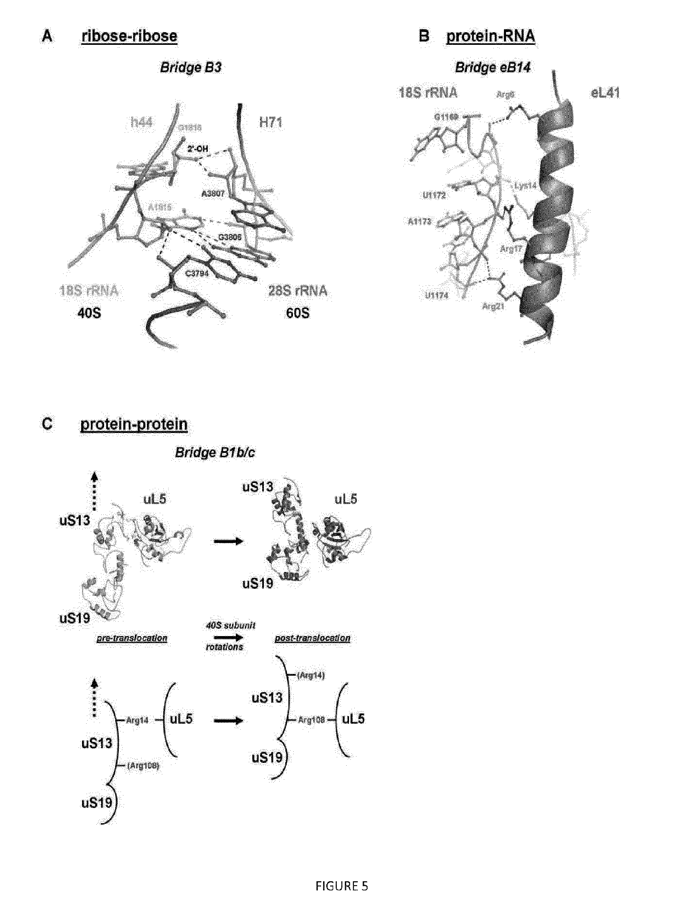

- Figure 5 represents molecular details of characteristic inter-subunit contacts. **(A)** Detail of bridge B3 highlighting ribose-ribose and ribose-nucleotide interactions between 28S rRNA helix H71 and 18S rRNA helix h44, mediated through the 2'-OH ribose moieties **(B)** Characteristic protein-RNA interaction between 18S rRNA region 1173 and the N-terminal helix of protein eL41 (bridge eB14) involving phosphate group interactions with positively charged residues (Arg and Lys) (C, D) Details of bridge B1 b/c which involves proteins uS13 and uL5 close to the central protuberance of the ribosome. The schematic drawing illustrates the remodelling of the inter-subunit interface that occurs at the atomic level between the pre- and post-translocation states (see also Fig. 3; present study and [6, 8] respectively)

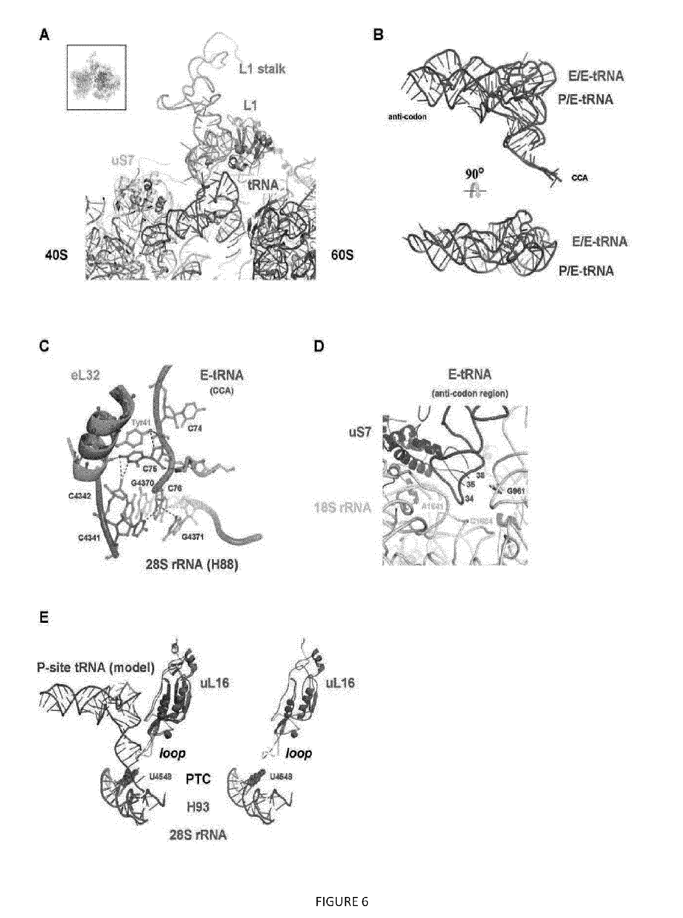

- Figure 6 represents molecular recognition by the human ribosome at the level of the E-site tRNA. **(A)** View into the E-site seen from the top (ribosomal subunits and the L1 stalk are annotated) **(B)** Comparison of the E-site tRNAs from the human and porcine ([6, 8] ribosome in the E/E- and P/E states (anticodon and CCA regions are labelled) **(C)** Details of the interactions between the CCA end region of the E-site tRNA and the 28S rRNA and protein eL32 of the 60S subunit, showing an intercalation of base C76 in between nucleotides G4370 and G4371, and a π-stacking of base C75 with Tyr41 of eL32. The antibiotic cycloheximide would clash with the CCA region of the tRNA **(D)** Contact regions of the E-site tRNA on the 40S subunit **(E)** Catalytic PTC region of the ribosome, highlighting the partial structural disorder of a region of protein uL16 (loop), and disorder of residue U4548 (28S rRNA) in the absence of P-site tRNA, while all other residues are well-ordered. This suggests that the P-site pocket is largely pre-defined while U4548 and the loop participate in tRNA accommodation.

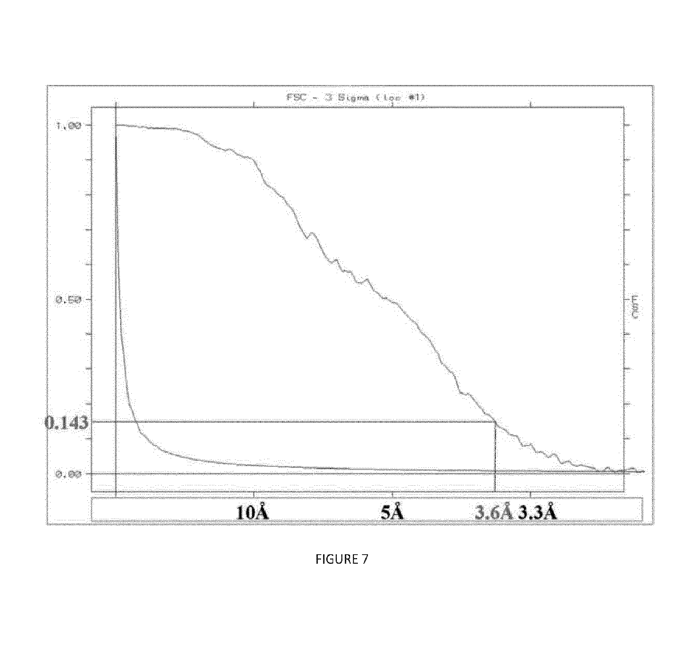

- Figure 7 represents the resolution estimation of the cryo-EM map as estimated from Fourier shell correlation according to the 0.143 criterion.

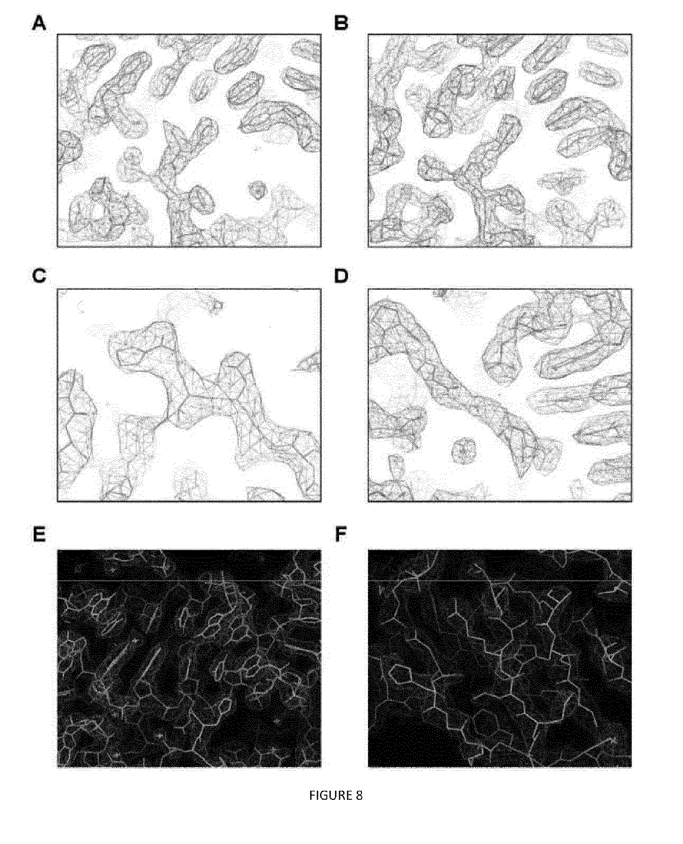

- Figure 8 represents the typical electron density map regions of the human ribosome structure. **(A, B, C, D, E, F)** These final electron density maps were obtained by combining experimental amplitudes derived from the cryo-EM map and phases calculated from the iteratively refined atomic model, as done in standard procedures in X-ray crystallography.

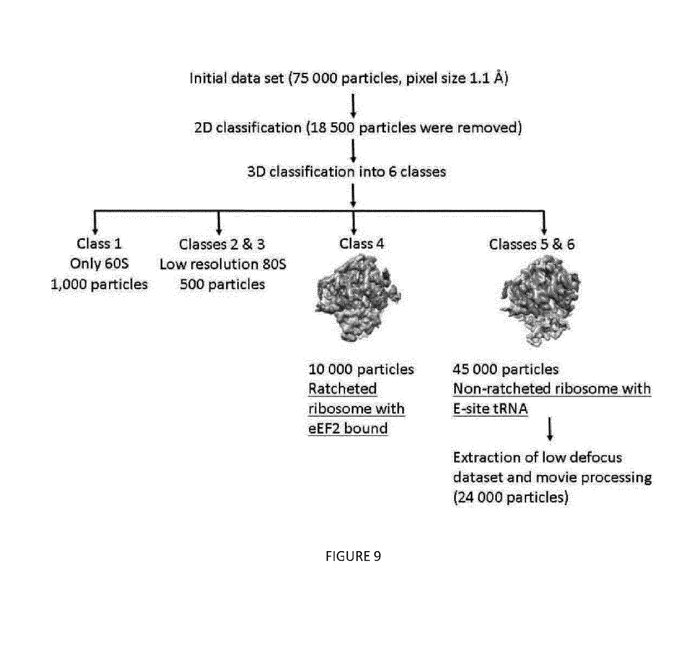

- Figure 9 represents particle sorting scheme by 3D classification.

### EXAMPLES

### Example 1: Material and Methods

### Purification

Human 80S ribosomes were prepared from HeLa cells, purified to homogeneity and characterized biochemically as described in the following. Briefly, the HeLa cells were lysed and the cytosolic extract was then loaded onto 30% sucrose cushion for overnight centrifugation. The ribosomal pellet was treated with puromycin before the final step of sucrose gradient and PEG precipitation. Concentration of purified 80S was calculated using the conversion 1 A₂₆₀ unit corresponds to 20 pmol of 80S ribosome.

### Step 1: Hela cell preparation

Hela cells are grown in suspension cultures (55x108 celles, about 6l) in Minimal Essential media Spinner Modification (S-MEM; Sigma Aldrich) supplemented with 7% newborn calf serum, 2mM Glutamine and 40µg/ml gentamycin at 37°C in 5% CO₂ environment. Once confluent, they are serum-starved for 6h to get a synchronized cell population.

### Step 2: Lysis and sucrose cushion

Cells are then lysed in reshly prepared lysis buffer containing 15mM Tris, pH 7.5, 0.5% NP40 (Sigma-Aldrich), 6mM MgCl2, 300mM NaCl, RNAsin (Promega). After 30 min of incubation on ice, the lysate is centrifuged at 12000g for 10min to remove debris, nuclei and mitochondria [60]. The supernatant is loaded on 30% sucrose cushion prepared in Buffer A and centrifuged for 16h at 115800g (50.2 Ti rotor) to get the crude ribosomal pellet (29,30). While loading the cushion, care must be taken to not disturb the sucrose, and ensure slow addition of the lysate to the 30% sucrose. This pellet is resuspended in Buffer B to homogeneity. The presence of nonresuspendeed particles in this solution can affect the next step, and these particles must be removed by a short centrifugation (10 min at 10000g). Only the supernatant is used for the next step.

### Step 3: Sucrose density gradient

Gradient preparation: SG 50 Gradient Maker (GE Healthcare) is used to make a linear gradient of 15-30%, wherein the higher % sucrose solution is loaded in the mixing chamber and the lower % sucrose solution is loaded in the other, allowing to mix slowly. The outlet is connected with a pump and sucrose is collected drop wise from the outlet.

The supernatant is treated with 1mM puromycin for 30min at 4°C [61] with intermittent mixing and loaded on 15-30% sucrose gradient prepared in Buffer A. The samples are centrifuged at 25000rpm for 11 h in a SW-28 rotor and fractions are collected from the bottom to top using an Econo Gradient Pump (Biorad) with an Econo UV Monitor (Biorad) and Fraction Collector. The sample absorbance is recorded using UV reader (Biorad) and the peak corresponding to 80S is pooled for PEG20K precipitation [1]. A final concentration of 7% PEG20K is added to the pooled fractions, incubated on ice for 10min and centrifuged at 17400g for 10 min. The pure ribosomal pellet is dissolved in resuspension buffer C and filtered using 0.22µm filters (Millipore) for further analysis or stored without filtration on ice for 7 days for crystallization. Snap freezing and storage is not advised. For concentration calculations, 1 A₂₆₀ unit corresponds to 20pmol of 80S ribosome.

### Data collection

2.5 µl of freshly prepared human 80S ribosomes, at 0.5 mg/ml, was applied to 300 mesh holey carbon Quantifoil 2/2 grids (Quantifoil Micro Tools, Jena, Germany), blotted with filter paper from both sides for half a second in the temperature- and humidity-controlled Vitrobot apparatus (FEI, Eindhoven, Netherlands, T = 20°C, humidity 95%, Blot Force 5, Blot time 0.5 sec) and vitrified in liquid ethane pre-cooled by liquid nitrogen. Data were collected on the in-house spherical aberration (Cs) corrected Titan Krios S-FEG instrument (FEI, Eindhoven, Netherlands) operating at 300 kV acceleration voltage and at a nominal underfocus of Δz = -0.6 to 4.5 µm using the back-thinned direct electron detector CMOS 4096 x 4096 camera and automated data collection with EPU software (FEI, Eindhoven, Netherlands). The microscope was carefully aligned as well as the Cs corrector. The Falcon II camera was calibrated at nominal magnification of 59 000 x. The calibrated magnification on the 14 µm pixel camera was 127 272 x resulting in 1.1A pixel size at the specimen level. Camera was set up to collect 3 frames (start form the second one) out of 17 possible, plus one total exposure image. Total exposure was 1 second with a dose of 60 e̅/Å² (or 3.5 e̅/Å² per frame).

### Image processing

Before particle picking stack alignment was performed, which included 3 frames and total exposure image (total 4 images in the stack). These 4 images in the stack were aligned by the whole image motion correction method described in [13]. Thereafter, an average image of whole stack was used to pick 75 000 particles semi automatically using EMAN2 Boxer [50], and the contrast transfer function of every image was determined using CTFFIND3 [51] in the RELION workflow [17]. Particle sorting was done by 3D classification using 6 classes starting from 75 000 particles, resulting in two dominant classes with 10 000 and 45 000 particles in rotated and non-rotated ribosome conformations respectively **(****Fig. 9****);** the subpopulation of the rotated ribosome probably contains elongation factor eEF2 and the ribosome is in a conformation similar to that of the previous lower-resolution human ribosome structure [6]. For the first steps of refinement, images were coarsened by 2 (C2 images) and 4 (C4-images) using EMAN2. The initial 2D classification as well as splitting of the different states of the ribosomes was done with the C4 images. Only once the states were clearly separated, uncoarsened (C1) data was used. During the last step of refinement, only close to focus data up to defocus 1.4 µm (about 24000 particles) was used. At the very last step the so called "movie processing" procedure as implemented in Relion1.3 version was used with only first 3 frames included in calculation as individual frames. A post-processing procedure implemented in RELION 1.2 [17] was applied to the final maps for appropriate masking, B-factor sharpening and resolution validation to avoid over-fitting [52]. In this procedure, the appropriate B-factor was determined according to [53]. The resolution was estimated in Relion at 0.143 FSC [53] and half-bit in Imagic (Fig. 7), indicating an average resolution of 3.6 Å [17]. After calculating local resolution values with ResMap [54] some regions are shown to reach ∼2.4 A resolution in the best cases, and up to 6 Å in the more flexible regions. Map interpretation was done using Chimera [55] and COOT [56] starting from the available atomic models [6, 8]. The atomic model was refined against the experimental map using Phenix [18] which included positional refinement, grouped B-factor refinement and simulated annealing. Figures prepared using the software Chimera [55] and Pymol (DeLano, 2006).

### Example 2 : Structure of the human ribosome 80S at the atomic level

### Structure determination

Human 80S ribosomes from HeLa cells were prepared using a recently established protocol [16] which allows obtaining highly homogenous samples of crystallizable quality, as also monitored through the observation of well-distributed particles in cryo-EM. In previous low-resolution cryo-EM single particle 3D reconstructions, we showed that these samples contain a tRNA in the E-site, but X-ray diffraction of the crystals was limited to ∼25 Å [16]. The combination of highly homogenous samples with on-site advanced single particle cryo-EM imaging techniques (high-resolution electron microscope equipped with a latest-generation high-sensitivity direct electron detector) now allowed us obtaining a structure at near-atomic resolution **(****Fig. 1A****).**

According to the "gold standard" Fourier shell correlation (FSC=0.143) criterion [17] the estimated resolution is ∼3.6 Å (Fig. 7). However, local resolution estimation **(****Fig. 2****)** shows that most regions are between 2.4 and 4.6 Å resolution with significant components at 2.9 Å, and only decrease to ∼6 Å in the more flexible regions at the periphery. In general, the 40S subunit appears more flexible resulting in some less well-defined regions at lower resolution as compared to the core of the 60S subunit which is structurally more stable with the exception of the L1 stalk region. The structure was interpreted in detail to derive an atomic model of the human ribosome obtained by extensive model building and structure refinement using Phenix [18]. For future complexes the current refined atomic model of the human 80S will be used, which will strongly increase the speed of the process of atomic model building of any new human 80S complex (whether with pharmaceutic ligands, proteins or RNA's). Most ribosomal proteins have about 95-100% conserved sequences in mammals except eL43, eL29, uL29, eL14 and eL6, which were rebuilt with the human sequences (ribosomal proteins follow the new nomenclature [15]). The atomic model was refined against the experimental cryo-EM map by iterative manual model building and restrained parameter refinement protocols, used in the field of X-ray crystallography [19] (real space refinement, positional refinement, grouped B-factor refinement and simulated annealing). We monitored the refinement process with R-factor values to avoid over-fitting, and refined the entire 80S structure at once (to ensure proper distance restraints between residues throughout the structure, notably at the subunit interface) by simulated annealing using parallel computing, rather than refining each protein subunit individually as for the recent porcine ribosome structure [8]. Next, we obtained maps with further improved features and resolution by combining the amplitudes of the Fourier-components of the cryo-EM map and the phases calculated from the atomic model. We combined this approach with a recent development in the field of X-ray crystallography called feature-enhanced maps (FEM, Afonine et al., Acta Cryst. D, in press) which robustly increases the interpretability of maps. Here, for the first time, we used FEM map calculations in the context of cryo-EM maps which for the well-ordered regions of the human 80S ribosome show resolution and features on side-chains that go beyond those of the original cryo-EM map itself. FEM maps were used for structure interpretation and fine-tuning the positions of side-chain conformations throughout the entire structure by manual model building, followed by simulated annealing protocols. Final electron density maps were calculated by combining phases of the refined atomic model and structure factors derived from the cryo-EM map, as is routinely done in the crystallography field, yielding even further improved maps (**Fig. 2G** and **Fig. 8**). This observation indicates that useful amplitudes are present in the cryo-EM data at a resolution beyond the average resolution estimated by Fourier-shell correlation. The final R-factor value of the refined structure (36%) compares well with that of crystal structures [18] showing that the atomic model is well refined compared to crystallography standards. The final atomic model (Fig. 1) comprises ∼220 000 atoms (excluding hydrogens) across the 5866 nucleotide residues and ∼11590 amino acids of the 80 proteins and the 4 rRNA's (28S, 5, 5.8 and 18S; excluding certain ES rRNA which are only partially visible at the periphery of the structure probably due to conformational heterogeneity). Protein residues show well-refined geometrical parameters (allowed regions 9.2%, preferred regions 83.2% in Ramachandran plots and only 7.7% of outliers which compares well with crystal structures).

### Description of the overall structure

The present structure provides a refined atomic model of the human 80S ribosome. It extends the completeness of the recent porcine ribosome structure at the level of several regions, such as the position of some rRNA segments (however, most rRNA expansion segments (ES) remained poorly defined). This includes the region of the L1 rRNA stalk and the L1 protein that could be completed; albeit at lower local resolution, this region is rather well ordered due to the presence of a tRNA in the E-site which was fully built in the atomic model. The structure provides unprecedented atomic details into functionally relevant key regions of the human ribosome including the PTC on the 60S ribosomal subunit, the decoding region on the 40S ribosomal subunit, the ribosomal subunit interface, the tRNA binding sites, the peptide exit channel, and potential ligand binding pockets. Interestingly, the α-sarcin-ricin loop region (rRNA helix H91) is entirely ordered even in the absence of ribosomal GTPases (including the A4605 region which is catalytically relevant for GTPase activation and the non base-paired G4600 located next to the G-domain of factors).

The overall structure shows the common landmarks of the 40S and 60S ribosomal subunits (**Fig. 1**). Compared to the yeast ribosome [1, 20], the human ribosome contains an additional protein, eL28 [6]. We observed additional densities corresponding to the C-terminus of uS2 (residues 209-222) and also redefined the globular structure of protein eS17 (residues 72-93) in the 40S subunit (**Fig. 2A&B**)**.** The present structure represents a pre-translocation state in which the 40S subunit is non-rotated (Fig. 3); this is a non-ratcheted state similar to the yeast ribosome [1] while the recent human and porcine ribosome structures exhibit essentially the same post-translocation conformation due to the presence of elongation factor eEF2 [6, 8]. This conformation is consistent with the presence of a tRNA molecule in the E-site and the movement of the L1 stalk towards the core of the ribosome. The L1 stalk rRNA interacts with the tRNA close to the elbow region, as observed earlier for the 70S ribosome [21-24]. In contrast to the previous human and porcine ribosome structures, the 40S subunit head shows a swivel movement away from the 60S which leads to a partial closure of the latch region (**Figs. 1** **&** **3A**). This could be related with the fact that the mRNA channel is entirely empty, i.e. it contains neither mRNA nor protein factors involved in ribosome starvation as found in yeast and previous human ribosome structures [1, 6]. In summary, the 40S subunit undergoes 3 characteristic types of conformational changes, (i) a universal rotation similar to the one described as "ratcheting" in bacterial ribosomes [25], (ii) a swivel movement of the 40S head and beak regions in the direction of the tRNA E-site, and (iii) a rotation of the 40S body around a vertical axis perpendicular to the classical rotation axis termed 'subunit rolling' (not observed in bacterial ribosomes, but recently described for a eukaryotic complex [26]; **Fig. 3A**). The subunit rolling leads to a slight opening of the ribosomal subunit interface (discussed below) on the side of the factor entry site where ribosomal GTPases bind. In this context it is also interesting to note that relatively large crevasses and gaps exist between the different structural domains of the 18S rRNA which could serve to accommodate conformational changes of the 40S throughout the translation process.

The transition from a pre-translocation to a post-translocation state induces several major rearrangements in the structure, highlighted by ribosomal proteins eL24 and eL19 (**Fig. 3B&C**) which constitute some of the characteristic eukaryote-specific bridges (eB12 and eB13, annotated in **Fig. 4**). The C-terminal part of eL24 relocates entirely and forms a new interaction site on the 40S subunit with ribosomal protein eS6 and 18S rRNA (**Fig. 3B**). In the case of eL19, a long protein helix protrudes from the 60S subunit to cross over to the 40S subunit. This is one of the rare 60S components (apart from the L1 region) that strongly change conformation upon subunit rotation. The C-terminal helix of protein eL19 is kinked in the pre-translocation state (and extends by another 7 residues) while it is straight in the post-translocation state (**Fig. 3C**). This conformational transition results in formation of salt bridges between the positively charged Arg172 and Arg176 side-chains of protein eL19 and the phosphate moieties of 18S rRNA nucleotides G909 and G910 (**Fig. 3C**).

### Inter-subunit interface

The present structure allows highlighting some of the characteristic inter-subunit contact sites in more detail with respect to the molecular interactions between the rRNA and protein residues involved. The interface comprises a series of contact regions that have been annotated (**Fig. 4**, summarized in **Table 1**) according to the nomenclature used for inter-subunit bridges of the yeast ribosome [1] extended by two new bridges eB15 and eB16 [6] but which are not seen in the present structure.

**Table 1**

| | **40S** | | **60S** | |
|---|---|---|---|---|
| | Human 80S | | Human 80S | |
| **B1a** | uS19 | Arg 13 | 28S | 1766 |
| **B1b/c** | uS13 | Asn17 | uL5 | Lys118 |
| | | Leu13 | | Asp114 |
| | | His11 | | Glu111 |
| | | Gln10 | | |
| **B2a** | 18S | 1706-1710 | 28S | 3765-3767 |
| | | 1826-1827 | | 3759-3762 |
| | | 1848-1852 | | |
| | | 1059 | | |
| **B2b** | 18S | 1848-1850 | 28S | 3699-3700 |
| | | 1069 | | |
| **B2c** | 18S | 1180 | 28S | 3694-3695 |
| **B2e** | 18S | 938-939 | eL43 | Arg 85 |
| **B3** | 18S | 1818 | 28S | 3628-3629 |
| | | 1815-1816 | | 3794-3796 |
| | | 1719-1721 | | 3805-3807 |
| **B4** | 18S | 1028-1029 | 28S | 1563-1565 |
| **B5** | 18S | 1731-1732 | 28S | 2884-2885 |
| **B6** | 18S | 1793 | L24e | Arg 47 |
| **B7a** | 18S | 970 | 28S | 3710-3711 |
| **B7b/c** | No | | | |
| **B8** | No | | No | |
| **eB8** | eS1 | Glu 224 | L8e | Thr263 |
| | | Leu 225 | | Hys 264 |
| | | Lys 227 | | |
| **eB11** | S8e | Arg 77 | 28S | 2893-2894 |
| | | Asn 88 | | 3598-3599 |
| | | Glu 89 | | 3610-3611 |
| | | Arg 92 | | 5016-5017 |
| | | Asp105 | | 5024 |
| | | Lys 124 | | 5026 |
| | | Gln 168 | | |
| | | Lys 170 | | |
| | | Arg 200 | | |
| | | Arg 205 | | |
| **eB12** | 18S | 871-873 | L19e | Arg162 |
| | | 908-910 | | Arg163 |
| | | 914 | | Lys167 |
| | | | | Arg170 |
| | | | | Arg 172 |
| | | | | Arg176 |
| **B13** | S6e | Asp151 | L24e | Arg47 |
| | | Asp 152 | | Glu 66 |
| | | Ser148 | | Lys70 |
| | | Leu147 | | Arg71 |
| | | Asn146 | | Arg73 |
| | | Arg131 | | Arg74 |
| | | Arg 132 | | Lys93 |
| | | Arg31 | | Arg94 |
| | | Met32 | | Asp95 |
| | | | | Gln96 |
| | | | | Lys97 |
| | | | | Arg101 |
| | | | | Arg105 |
| | | | | Ala108 |
| | | | | Lys124 |
| | 18S | 1779-1781 | | |
| | | 1783 | | |
| | | 1746-1747 | | |
| | | 323 | | |
| **eB14** | 18S | 1719 | L41e | Arg2 |
| | | 1171-1175 | | Lys4 |
| | | 1180 | | Arg6 |
| | | 1182-1183 | | Lys7 |
| | | 1841-1852 | | Lys8 |
| | | 1706 | | Arg9 |
| | | | | Arg11 |
| | | | | Arg 12 |
| | | | | Lys14 |
| | | | | Arg15 |
| | | | | Arg17 |
| | | | | Arg18 |
| | | | | Arg21 |

The core of the contact interface is formed by helix H69 (28S rRNA, A3766 region) from the 60S subunit which interacts with helix h44 (18S rRNA, G1849 region; **Fig. 4**) to form bridges B2a and B2b, close to the universal rotation axis (**Fig. 3A**) around which the ribosomal subunits rotate forward and backwards during the tRNA translocation process as seen in prokaryotic and eukaryotic ribosomes [24, 25, 27, 28]. Among the known inter-subunit bridges, B7b/c and B8 are not observed in the present human 80S ribosome (not including contact-mediating ions such as Mg²⁺ or Os) while the bridge eB8 contact is mediated by eS1 and eL8, in stark contrast with the yeast 80S ribosome [1] where eL8 is replaced by eL43. Also, there is an additional B2e bridge present as observed in [26]. It is remarkable that a noticeable gap exists between the 40S and 60S subunits in the region below the factor binding site, which is related possibly with the vertical rotation of the 40S subunit and a slight opening of the interface (**Fig. 3A**). In contrast to other contact regions there are only few densities observed in the inter-subunit space which could be ions such as Mg²⁺. In general, four contact types are observed: ribose-ribose, ribose-phosphate, ribose-base, protein-base and protein-protein interactions (**Fig. 5**). In the following we highlight some of the most prominent examples of inter-subunit contact regions in the human ribosome.

A particularly interesting contact type is the interaction mediated by ribose moieties, with the 2'-OH groups of the rRNA backbone sugar moieties playing a key role rather than the charged phosphate groups of the rRNA backbone (**Fig. 5A**). The 2'-OH groups are offset, leading to a bifurcated hydrogen-bonding pattern. This pattern is distinct from sugar-edge (ribose-base) interactions [29] and somehow similar to that of protein ß-sheets involving the carbonyl backbone [30-32]. This leads to a characteristic ribose-ribose interface involving mostly hydroxyl groups of the sugar moieties, as illustrated by the interactions of the 2'-OH groups of nucleotides A1719, A1815 and G1816 (18S rRNA helix h44) with the 2'-OH groups of nucleotides G3806, A3807, C3794 and A3795 (28S rRNA helix H71; **Fig. 5A**). Similar ribose-ribose interactions exist between ribose moieties in the subunit interface of bacterial ribosomes [33-35]. These ribose-ribose contact regions contrast with other regions of the interface in which a significant amount of interactions occur through Mg²⁺-mediated phosphate-phosphate electrostatic interactions, while the bridges located more at the periphery operate through direct hydrogen bonding rather than being mediated by Mg²⁺ (e.g. uS13-uL5, or the eukaryote-specific eL19 and the 18S rRNA 910 region; **Figs. 3B&C**). This difference could explain the observation that the biochemical stability of eukaryotic ribosomes is much less Mg²⁺-dependent as compared to bacterial ribosomes, and much more dependent on K+-ions, consistent with the idea that potassium is required for inter-subunit stability while magnesium ensures proper rRNA folding [16, 36-38]. Characteristic protein-RNA interactions exist for example between 18S rRNA region 1173 and the N-terminal helix of protein eL41 and involve phosphate group interactions with positively charged residues (Arg and Lys; **Fig. 5B**). Another example of interface remodelling are regions of the ribosomal subunit interface in which rRNA ribose and Mg-mediated contacts sites are also seen to switch positions upon rotation of the ribosomal subunits. This involves rearrangement of the interface and Mg-ion repositioning (e.g. bridges B2b, 1848 nucleotide region of 18S rRNA, and phosphate backbone of nucleotide 1070; solvent channels with residual densities for potential ions are found right below the E-site tRNA binding site).

Due to the conformational difference of the structure as compared to previous human and porcine ribosome structures numerous contact sites are shifted or completely rearranged. Apart from the two examples of bridges eB12 and eB13 described above that involve major conformational changes of the involved proteins, another prominent example is that of bridge B1 b/c (**Fig. 5C**). This contact is located between the central protuberance of the 60S and the 40S head and involves ribosomal proteins uL5 and uS13 (**Fig. 5C**). Rather than operating through large conformational changes of parts of the proteins, this bridge rearranges through a protein shift which is amplified at the periphery of the 40S subunit when rotating. Upon subunit rotation and 40S head swivelling a shift of ∼23Å of ribosomal protein uS13 occurs, leading to a complete remodelling of the molecular interactions between uS13 and uL5. In the pre-translocation state, the benzoyl moiety of Tyr119 (uL5) make a hydrophobic contact with the carbon part of the side chains of Arg14 and Ile12 (uS13). By contrast, in the post-translocation state as observed in the previous human and porcine ribosome structures (which have eEF2 bound) the same residues of uL5 interact with residues Asn105 and Arg108 of uS13 (see schematic drawing **Fig. 5D**); the large shift allows additional interactions between uL5 and uS19 to be formed. Similar rearrangements have been observed in the yeast and E. *coli* ribosomes [1, 33, 39].

### tRNA binding sites and molecular recognition of the E-site tRNA

The present human 80S ribosome contains a co-purified tRNA in the E-site which is well-defined in the cryo-EM map and appears to stabilize the L1 stalk (**Fig. 6A**) as also observed in the previous human 80S ribosome structure [6]. Comparison with other E-site tRNA structures shows that its conformation is different in the anti-codon loop regions of the decoding stem, while it is similar at the CCA end region of the acceptor stem (**Fig. 6B**). The tRNA is found in an E/E state consistent with the pre-translocation state of the ribosome, while it is observed in a P/E hybrid state in the porcine ribosome [8, 40, 41]. The E-site tRNA interacts with several regions of the ribosome. On the 60S subunit, interactions are seen with the L1 stalk which is oriented inwards, and with the L1 protein at the level of the tRNA elbow. The L1 stalk rRNA interacts with the tRNA close to the elbow region and at the top of decoding stem **(****Fig. 6A****).** The CCA end exhibits specific interactions in a ligand pocket formed by G4370 and G4371 of the 28S rRNA and ribosomal protein eL32 (Fig. 6C). A76 of the tRNA intercalates between bases G4370 and G4371, while C75 forms π-stacking interactions with Tyr41 of eL32 (**Fig. 6C**); additional interactions are seen between the 4360 region, A296 and Tyr41 and Lys30 of eL32. The existence of molecular recognition events in the E-site binding pocket is an interesting observation considering that the tRNA behaves as a reaction product which maintains specific interactions with the enzyme, after the transfer of the nascent peptide to the P-site tRNA during the translation process. These specific interactions are present although the tRNA probably is a mixture of co-purified tRNAs, suggesting that these interactions are common to all tRNA, consistent with the universal conservation of the CCA end sequence [42]. The binding pocket of the tRNA CCA end overlaps with the binding site of the antibiotic cycloheximide known from the yeast ribosome [43]. On the 40S side the E-site tRNA decoding stem fits into a pocket formed by protein uS7 and bases G961, A1641 and C1684 of the 18S rRNA (**Fig. 6D**). While details of the ribosome interactions with the anti-codon cannot be resolved due to the mixed nature of the co-purified tRNA, their presence suggests that interactions with the mRNA are partially released in the E-site as seen in 70S crystal structures [44].

The A- and P-sites are empty in the present structure, but nevertheless the two binding pockets are precisely preformed for molecular recognition of the tRNA molecules. This is evident from the fact that most rRNA and protein moieties constituting the tRNA pockets are structurally well-ordered including the vast majority of side-chains. Exceptions to this are A4548 (28S rRNA helix H93, part of the PTC, corresponds to A2602 in *E. coli*) and the central part of protein uL16 that forms a loop (residues 101-117) which are essentially disordered in the P-site (while the C-terminal part of uL16 located in the A-site is fully defined; **Fig. 6E**). This suggests that these components of the P-site pocket actively accommodate the tRNA acceptor stem and CCA end region upon binding and become structurally ordered (in the presence of a P-site tRNA the loop interacts with bases 1 and 2 of the tRNA) [45].

The structural stabilization upon substrate binding also applies to helix H69 on which the P-site tRNA decoding stem lodges, and to U4452 in the PTC at the peptide channel entry. H69 shows some structural disorder, while 18S rRNA helix h44 with which it forms inter-subunit bridge B2a is well-ordered. Thus, P-site tRNA binding is likely to reinforce inter-subunit interactions across this bridge through the stabilization of H69, consistent with the idea that P-site tRNA binding stabilizes the fully assembled 80S complex which is required for translation initiation [46-48]). Similarly, nucleotide U4452 of 28S rRNA at the entry of the peptide exit channel is disordered in the absence of growing peptide, while all side-chains forming the peptide channel through the 60S subunit are well-ordered. U4452 is located at a potential branching point of the peptide channel, suggesting that this residue could serve as a gate-keeper to correctly orient the early growing nascent peptide.

Regarding the A-site, all ribosomal residues are defined including nucleotides U4501, U4500 and G4499 (helix H92 of 28S rRNA) are well-defined and pre-oriented for base-pair interactions respectively with the complementary C74, C75 and A76 nucleotides of the tRNA, with U4438 (H89 of 28S rRNA) pre-oriented for recognition of A73 of the tRNA. On the 40S subunit, the decoding centre is entirely pre-formed including most side-chains. The universally conserved nucleotide residues A1824 and A1825 of 18S rRNA helix h44 (A1492 & A1493 in E. *coli* numbering) are not well resolved to designate their position as flipped-in/out positions which is known to be important for decoding [49].

### List of references

1. Ben-Shem, A., et al., The structure of the eukaryotic ribosome at 3.0 A resolution. Science, 2011. 334(6062): p. 1524-9.
2. Rabl, J., et al., Crystal structure of the eukaryotic 40S ribosomal subunit in complex with initiation factor 1. Science, 2011. 331(6018): p. 730-6.
3. Klinge, S., et al., Crystal structure of the eukaryotic 60S ribosomal subunit in complex with initiation factor 6. Science, 2011. 334(6058): p. 941-8.
4. Spahn, C.M., et al., Cryo-EM visualization of a viral internal ribosome entry site bound to human ribosomes: the IRES functions as an RNA-based translation factor. Cell, 2004. 118(4): p. 465-75.
5. Boehringer, D., et al., Structure of the hepatitis C virus IRES bound to the human 80S ribosome: remodeling of the HCV IRES. Structure, 2005. 13(11): p. 1695-706.
6. Anger, A.M., et al., Structures of the human and Drosophila 80S ribosome. Nature, 2013. 497(7447): p. 80-5.
7. Chandramouli, P., et al., Structure of the mammalian 80S ribosome at 8.7 A resolution. Structure, 2008. 16(4): p. 535-48.
8. Voorhees, R.M., et al., Structure of the Mammalian Ribosome-Sec61 Complex to 3.4 A Resolution. Cell, 2014.
9. Amunts, A., et al., Structure of the yeast mitochondrial large ribosomal subunit. Science, 2014. 343(6178): p. 1485-9.
10. Greber, B.J., et al., The complete structure of the large subunit of the mammalian mitochondrial ribosome. Nature, 2014. 515(7526): p. 283-6.
11. Brown, A., et al., Structure of the large ribosomal subunit from human mitochondria. Science, 2014. 346(6210): p. 718-22.
12. Li, X., et al., Influence of electron dose rate on electron counting images recorded with the K2 camera. J Struct Biol, 2013. 184(2): p. 251-60.
13. Li, X., et al., Electron counting and beam-induced motion correction enable near-atomic-resolution single-particle cryo-EM. Nat Methods, 2013. 10(6): p. 584-90.
14. Bai, X.C., et al., Ribosome structures to near-atomic resolution from thirty thousand cryo-EM particles. Elife, 2013. 2: p. e00461.
15. Ban, N., et al., A new system for naming ribosomal proteins. Curr Opin Struct Biol, 2014. 24: p. 165-9.
16. Khatter, H., et al., Purification, characterization and crystallization of the human 80S ribosome. Nucleic Acids Res, 2014. 42(6): p. e49.
17. Scheres, S.H., RELION: implementation of a Bayesian approach to cryo-EM structure determination. J Struct Biol, 2012. 180(3): p. 519-30.
18. Afonine, P.V., et al., Towards automated crystallographic structure refinement with phenix.refine. Acta Crystallogr D Biol Crystallogr, 2012. 68(Pt 4): p. 352-67.
19. DeLaBarre, B. and A.T. Brunger, Considerations for the refinement of low-resolution crystal structures. Acta Crystallogr D Biol Crystallogr, 2006. 62(Pt 8): p. 923-32.
20. Ben-Shem, A., et al., Crystal structure of the eukaryotic ribosome. Science, 2010. 330(6008): p. 1203-9.
21. Fei, J., et al., Coupling of ribosomal L1 stalk and tRNA dynamics during translation elongation. Mol Cell, 2008. 30(3): p. 348-59.
22. Cornish, P.V., et al., Following movement of the L1 stalk between three functional states in single ribosomes. Proc NatI Acad Sci U S A, 2009. 106(8): p. 2571-6.
23. Feng, S., Y. Chen, and Y.G. Gao, Crystal structure of 70S ribosome with both cognate tRNAs in the E and P sites representing an authentic elongation complex. PLoS One, 2013. 8(3): p. e58829.
24. Valle, M., et al., Locking and unlocking of ribosomal motions. Cell, 2003. 114(1): p. 123-34.
25. Frank, J. and R.K. Agrawal, A ratchet-like inter-subunit reorganization of the ribosome during translocation. Nature, 2000. 406(6793): p. 318-22.
26. Budkevich, T.V., et al., Regulation of the mammalian elongation cycle by subunit rolling: a eukaryotic-specific ribosome rearrangement. Cell, 2014. 158(1): p. 121-31.
27. Budkevich, T., et al., Structure and dynamics of the mammalian ribosomal pretranslocation complex. Mol Cell, 2011. 44(2): p. 214-24.
28. Chen, J., et al., Coordinated conformational and compositional dynamics drive ribosome translocation. Nat Struct Mol Biol, 2013. 20(6): p. 718-27.
29. Leontis, N.B. and E. Westhof, Geometric nomenclature and classification of RNA base pairs. RNA, 2001. 7(4): p. 499-512.
30. Klaholz, B. and D. Moras, C-H...O hydrogen bonds in the nuclear receptor RARgamma--a potential tool for drug selectivity. Structure, 2002. 10(9): p. 1197-204.
31. Liu, A., et al., NMR detection of bifurcated hydrogen bonds in large proteins. J Am Chem Soc, 2008. 130(8): p. 2428-9.
32. Jiang, L. and L. Lai, CH...O hydrogen bonds at protein-protein interfaces. J Biol Chem, 2002. 277(40): p. 37732-40.
33. Schuwirth, B.S., et al., Structures of the bacterial ribosome at 3.5 A resolution. Science, 2005. 310(5749): p. 827-34.
34. Cate, J.H., et al., X-ray crystal structures of 70S ribosome functional complexes. Science, 1999. 285(5436): p. 2095-104.
35. Yusupov, M.M., et al., Crystal structure of the ribosome at 5.5 A resolution. Science, 2001. 292(5518): p. 883-96.
36. Shenvi, C.L., et al., Accessibility of 18S rRNA in human 40S subunits and 80S ribosomes at physiological magnesium ion concentrations--implications for the study of ribosome dynamics. RNA, 2005. 11 (12): p. 1898-908.
37. Moore, M.N. and L.L. Spremulli, Effects of cations and cosolvents on eukaryotic ribosomal subunit conformation. Biochemistry, 1985. 24(1): p. 191-6.
38. Sperrazza, J.M., D.W. Russell, and L.L. Spremulli, Reversible dissociation of wheat germ ribosomal subunits: cation-dependent equilibria and thermodynamic parameters. Biochemistry, 1980. 19(6): p. 1053-8.
39. Zhang, W., J.A. Dunkle, and J.H. Cate, Structures of the ribosome in intermediate states of ratcheting. Science, 2009. 325(5943): p. 1014-7.
40. Dunkle, J.A., et al., Structures of the bacterial ribosome in classical and hybrid states of tRNA binding. Science, 2011. 332(6032): p. 981-4.
41. Tourigny, D.S., et al., Elongation factor G bound to the ribosome in an intermediate state of translocation. Science, 2013. 340(6140): p. 1235490.
42. Selmer, M., et al., Structure of the 70S ribosome complexed with mRNA and tRNA. Science, 2006. 313(5795): p. 1935-42.
43. Garreau de Loubresse, N., et al., Structural basis for the inhibition of the eukaryotic ribosome. Nature, 2014. 513(7519): p. 517-22.
44. Jenner, L., et al., Messenger RNA conformations in the ribosomal E site revealed by X-ray crystallography. EMBO Rep, 2007. 8(9): p. 846-50.
45. Rhodin, M.H. and J.D. Dinman, A flexible loop in yeast ribosomal protein L11 coordinates P-site tRNA binding. Nucleic Acids Res, 2010. 38(22): p. 8377-89.
46. Yamamoto, H., et al., Structure of the mammalian 80S initiation complex with initiation factor 5B on HCV-IRES RNA. Nat Struct Mol Biol, 2014. 21(8): p. 721-7.
47. Hinnebusch, A.G., The scanning mechanism of eukaryotic translation initiation. Annu Rev Biochem, 2014. 83: p. 779-812.
48. Pestova, T.V., et al., Molecular mechanisms of translation initiation in eukaryotes. Proc NatI Acad Sci USA, 2001. 98(13): p. 7029-36.
49. Demeshkina, N., et al., A new understanding of the decoding principle on the ribosome. Nature, 2012. 484(7393): p. 256-9.
50. Ludtke, S.J., P.R. Baldwin, and W. Chiu, EMAN: semiautomated software for high-resolution single-particle reconstructions. J Struct Biol, 1999. 128(1): p. 82-97.
51. Mindell, J.A. and N. Grigorieff, Accurate determination of local defocus and specimen tilt in electron microscopy. J Struct Biol, 2003. 142(3): p. 334-47.
52. Chen, S., et al., High-resolution noise substitution to measure overfitting and validate resolution in 3D structure determination by single particle electron cryomicroscopy. Ultramicroscopy, 2013. 135: p. 24-35.
53. Rosenthal, P.B. and R. Henderson, Optimal determination of particle orientation, absolute hand, and contrast loss in single-particle electron cryomicroscopy. J Mol Biol, 2003. 333(4): p. 721-45.
54. Kucukelbir, A., F.J. Sigworth, and H.D. Tagare, Quantifying the local resolution of cryo-EM density maps. Nat Methods, 2014. 11(1): p. 63-5.
55. Pettersen, E.F., et al., UCSF Chimera--a visualization system for exploratory research and analysis. J Comput Chem, 2004. 25(13): p. 1605-12.
56. Emsley, P., et al., Features and development of Coot. Acta Crystallogr D Biol Crystallogr, 2010. 66(Pt 4): p. 486-501.
57. Sohmen et al., Enhanced SnapShot : Antibiotic inhibition of protein synthesis II. Cell, 2009. 139(1): p. 212-212.e1. doi: 10.1016/j.cell.2009.08.009.
58. Sohmen et al., SnapShot : Antibiotic inhibition of protein synthesis I. Cell, 2009. 138(6): p. 1248.e1. doi: 10.1016/j.cell.2009.08.001.
59. Gold et al., Development and validation of a genetic algorithm for flexible docking. J. Mol. Biol., 1997. 267(3): 727-48.
60. Belin et al., Purification of ribosomes from human cell lines. Curr. Protoc. Cell Biol., 2010. Chapter 3: Unit 3.40.
61. Blobel et al., Dissociation of mammalian polyribosomes into subunits by puromycin. Proc. NatI. Acad. Sci. USA, 1971. 68: p. 390-4.
62. Miranker and Karplus, Functionality Maps of Binding Sites: A Multiple Copy Simultaneous Search Method. Proteins, 1991. 11: p. 29-34
63. Goodsell and Olsen, Automated Docking of Substrates to Proteins by Simulated Annealing. Proteins, 1990. 8: p. 195-202

## Claims

1. Use of a 3D structural representation at atomic resolution of the human 80S ribosome obtained by a method comprising the steps of:
**a)** providing highly homogenous human 80S ribosomes;
**b)** characterizing the structural representation at atomic resolution of the human 80S ribosome using a high-resolution cryo electron microscope equipped with a high-sensitivity direct electron detector;
**c)** high-resolution image processing and 3D reconstruction of the single particle images obtained in step b) with a resolution in the range of 1.8-3.2 Å;
in a drug design method or a screening method.

2. Method of using the 3D structural representation at atomic resolution of the human 80S ribosome obtained by the method as defined in claim 1, to identify a molecule of interest that can selectively bind said ribosome.

3. Method of using according to claim 2, wherein the molecule of interest is chosen from the group consisting in anti-cancer compounds, anti-infectious compounds, and ribosome-associated cellular and extra-cellular protein and RNA factors.

4. A screening method for the identification of a compound that selectively binds the human 80S ribosome, comprising the steps of:
**a)** providing highly homogenous human 80S ribosomes;
**b)** providing a test compound;
**c)** contacting the human 80S ribosomes with the test compound;
**d)** characterizing the structural representation of the complex formed in step c) using a high-resolution cryo electron microscope equipped with a high-sensitivity direct electron detector;
**e)** high-resolution image processing and 3D reconstruction of the single particle images obtained in step d) with a resolution in the range of 1.8-3.2 Å.

5. The screening method of claim 4, further comprising a step of **f)** assaying the interaction between the test compound and the human 80S ribosome.

6. The screening method of claim 4 or 5, wherein the test compound is chosen from the group in anti-cancer compounds, anti-infectious compounds, and ribosome-associated cellular and extra-cellular protein and RNA factors.

7. A computer-based method for identifying a ligand for the human 80S ribosome, comprising the steps of:
**a)** providing a 3D structural representation of the human 80S ribosome obtained by the method as defined in claim 1; and
**b)** using the computer to apply structure-based drug design techniques to the structural representation.

8. The method of claim 7 further comprising at least one step of docking.

9. The method of claim 8, wherein the docking step screens members of a structural library of potential ligands.

10. The method of anyone of claims 7 to 9, comprising the further step of c) providing a compound identified by said-structure-based drug design techniques; and d) contacting said compound with the human 80S ribosome and assaying the interaction between them.

11. A method for obtaining a 3D structural representation at atomic resolution of the human 80S ribosome, said method comprising the steps of:
**d)** providing highly homogenous human 80S ribosomes;
**e)** characterizing the structural representation at atomic resolution of the human 80S ribosome using a high-resolution cryo electron microscope equipped with a high-sensitivity direct electron detector;
**f)** high-resolution image processing and 3D reconstruction of the single particle images obtained in step b) with a resolution in the range of 1.8-3.2 Å.

12. 3D structural representation at atomic resolution of the human 80S ribosome obtained by the method of claim 11 and having a resolution in the range of 1.8-3.2 Å.

13. Use of the 3D structural representation at atomic resolution of the human 80S ribosome according to claim 12 into a drug design method or a screening method.
